# EUROPEAN PATENT APPLICATION

(11) **EP 4 843 515 A1**
(43) Date of publication of application: **25.11.2026**
(21) Application number: 26179683.3
(22) Date of filing: 19.05.2026
(51) Int. Cl.: A61B 17/072, A61B 34/00, A61B 34/30, A61B 17/00, A61B 90/00

(54) **TECHNOLOGIES FOR DETERMINING AND SCHEDULING TRANSECTION PAUSES IN A FIRING PHASE OF A SURGICAL INSTRUMENT**

(30) Priority: 19.05.2025 US 202519212569
(71) Applicant: Cilag GmbH International, 6300 Zug (CH)
(72) Inventor: OVERMYER, Mark D, 6300 Zug (CH); DICKSON, Heather, 6300 Zug (CH); HILL, Matthew, 6300 Zug (CH)
(74) Representative: Carpmaels & Ransford LLP

(57) **Abstract**

Technologies for determining and scheduling transection pauses in a firing phase of a surgical instrument includes a surgical stapler and a control system. The surgical stapler includes an end effector having a jaw assembly controllable to grasp and compress tissue captured in the jaw assembly. The control system is configured to determine a total amount of work completed by the jaw assembly to compress the tissue and determine a number of transection pauses to be performed by the end effector during a firing phase of the surgical stapler based on the determined total amount of work performed by the end effector to compress the tissue. The control system may also determine a location for each transection pause relative to peak firing force of a firing force profile of the end effector during the firing phase.

## Description

### CROSS-REFERENCE TO RELATED APPLICATIONS

The present application claims benefit of and priority to U.S. Non-Provisional Patent Application No. 19/212,569, filed May 19, 2025, with the United States Patent & Trademark Office, the contents of which are hereby incorporated by reference in its entirety.

### TECHNICAL FIELD

The present disclosure relates generally to surgical instruments and, more particularly, to surgical stapling and cutting instruments and associated staple cartridges for use with the surgical instruments to facilitate the cutting and stapling of patient tissue.

### BACKGROUND

Surgical procedures often involve the use of various surgical instruments to assist a surgeon in the performance of the corresponding procedure. Recently, minimally invasive surgical (MIS) instruments have gained preference over traditional "open" surgical devices as the use of MIS instruments can reduce post-operative recovery time and associated tissue scarring. Endoscopy and laparoscopy are common types of MIS procedures in which a tube or trocar is inserted into natural or surgically-made openings (e.g., incisions) of the patient, depending on the particular procedure. The tube or trocar is then used to facilitate introduction of associated surgical instruments into the patient's body (e.g., the patient's abdominal cavity) to treat internal tissue of the patient.

One type of surgical instrument usable in MIS procedures is a surgical stapler, sometimes referred to as an "endocutter." A typical surgical stapler includes an elongated shaft to facilitate use of a trocar and an end effector located at a distal end of the elongated shaft. The elongated shaft facilitates the use of the surgical stapler with a trocar to access the patient's anatomical environment (e.g., the patient's abdominal cavity) and may include one or more articulation joints to increase the usability of the surgical stapler in the anatomical environment. Similarly, the end effector may be attached to the elongated shaft via an articulation joint to allow the end effector to be positioned as desired by the surgeon. A typical end effector of a surgical stapler includes a pair of jaws movable between an open position and a closed position to facilitate the grasping of tissue within the jaws. One of the jaws is embodied as a cartridge jaw and is configured to receive a staple cartridge, and the other jaw is embodied as an anvil jaw configured to provide a surface on which the staples are formed. The end effector also includes a cutting element (typically referred to as a "knife"), which is translated distally along the end effector during a firing phase of the surgical stapler to transect tissue presently grasped by the end effector. As the knife advances along the end effector, staples contained in the staple cartridge are progressively ejected to thereby seal opposing sides of the transected tissue.

Surgical staplers may be embodied as independent, hand-held devices or configured to be coupled to a robotic surgery system. In hand-held embodiments, the surgical stapler may include a handle and an associated trigger, which is operable by a surgeon to control the end effector and the ejection of the staples from the staple cartridge. In robotic embodiments, the surgical stapler is configured to be mounted to a robotic arm of a robotic manipulator, which is controllable by the surgeon via a remote control console. For example, the control console may include various input devices that can be grasped and manipulated by the surgeon to cause movement and firing of the surgical stapler.

In some typical surgical staplers, the surgical stapler may institute a pause during the firing phase due to difficulties in compression of the tissue. During the pause, the advancement of the knife of the surgical stapler may be temporarily halted. In some cases, however, the firing pauses may randomly occur or occur due to factors unknown by the surgeon, which can lead to uncertainty of the surgeon in the success of the firing phase.

### SUMMARY

According to an aspect of the present disclosure, a surgical system may include a surgical stapler and a control system. The surgical stapler may include an end effector having a jaw assembly movable from an open state to a closed state to compress tissue captured within the jaw assembly. When in the closed state, the surgical stapler may be operable to perform a firing phase to transect and staple the tissue captured within the jaw assembly. The control system may be configured to control operation of the surgical stapler and may be configured to determine a total amount of work completed by the jaw assembly to compress the tissue and determine a number of transection pauses to be performed by the end effector during the firing phase of the surgical stapler based on the determined total amount of work performed by the end effector to compress the tissue.

According to another aspect of the present disclosure, a control system for controlling operation of a surgical stapler including an end effector having a jaw assembly moveable from an open state to a closed state to compress tissue captured within the jaw assembly may include a torque sensor, a rotary sensor, and a controller. The torque sensor may be configured to generate torque sensor data indicative of an amount of torque of a motor configured to control the jaw assembly of the surgical stapler. The rotary encoder may be configured to generate rotation sensor data indicative of the rotational angle of the motor. The controller configured to determine a total amount of work completed by the jaw assembly to compress the tissue based on the torque sensor data and the rotation sensor data and to determine, based on the determined total amount of work performed by the end effector to compress the tissue, a number of transection pauses to be performed by the end effector during a firing phase of the surgical stapler to transect and staple the tissue captured within the jaw assembly.

According to a further aspect of the present disclosure, a robotic manipulator may include a robotic arm and a control system. The robotic arm may include an arm interface configured to mate with a tool interface of a surgical stapler to couple the surgical stapler to the robotic arm. The control system may be configured to control operation of the surgical stapler when the surgical stapler is coupled to the robotic arm to move a jaw assembly of an end effector of the surgical stapler from an open state to a closed state to compress tissue captured within the jaw assembly, determine a total amount of work completed by the jaw assembly to compress the tissue, and determine, based on the determined total amount of work performed by the end effector to compress the tissue, a number of transection pauses to be performed by the end effector during a firing phase of the surgical stapler to transect and staple the tissue captured within the jaw assembly.

According to an additional aspect of the present disclosure, a method for controlling operation of a surgical staple may include determining, by a control system, a total amount of work completed by a jaw assembly of an end effector of the surgical stapler to compress tissue captured within the jaw assembly and determining, by the control system, a number of transection pauses to be performed by the end effector during a firing phase of the surgical stapler based on the determined total amount of work performed by the end effector to compress the tissue. The method may also include controlling, by the control system, the surgical stapler to perform a firing phase of the end effector of the surgical stapler to transect and staple the tissue captured within the jaw assembly, wherein to perform the firing phase includes to pause transection of the tissue by the end effector based on the number of transection pauses.

In some embodiments, to determine the total amount of work performed by the jaw assembly may include to determine, while the jaw assembly compresses the tissue, an amount of torque of a motor configured to control the jaw assembly; determine, while the jaw assembly compresses the tissue, a rotational angle of the motor; and determine the total amount of work performed by the jaw assembly to compress the tissue based on the determined amount of torque of the motor and the rotational angle of the motor. Additionally, in some embodiments, the control system may include a torque sensor configured to generate sensor data indicative of the torque of the motor and a rotary encoder configured to generate sensor data indicative of the rotational angle of the motor.

In some embodiments, the end effector may include a knife movable to transect the tissue compressed by the jaw assembly during the firing phase of the end effector. Additionally, movement of the knife is halted during each transection pause. Additionally, in some embodiments, to determine the number of transection pauses to be performed by the end effector during the firing phase of the surgical stapler may include to determine a number of reference work increments completed within the total amount of work completed by the jaw assembly to compress the tissue and to increment the number of transection pauses for each reference work increment completed. In such embodiments, the reference work increment may be defined as 0.25 Joules.

Additionally, in some embodiments, the control system may be further configured to determine a position for each transection pause on a firing force profile of the end effector during the firing phase. The firing force profile of the end effector may be based on a historical firing force profile of the end effector. In some embodiments, to determine the position for each transection pause may include to space each transection pause in reference to a peak firing force value of the firing force profile. For example, to space each transection pause in reference to the peak firing force value may include to evenly space each transection pause from the peak firing force value of the firing force profile.

Furthermore, in some embodiments, the control system may further be configured to display a simulated transection profile to an operator of the surgical stapler prior to the firing phase of the surgical stapler. In such embodiments, the simulated transection profile may be indicative of a position of a knife of the end effector throughout the firing phase and includes indicia of the number and position of each transection pause on the simulated transection profile. Additionally, movement of the knife is halted during each transection pause.

In some embodiments, the control system may be further configured to communicate indicia of the number of transection pauses to an operator of the surgical stapler. For example, to communicate indicia of the number of transection pauses may include to display a pause count indicator to the operator. The pause count indicator may be indicative of the number of transection pauses. Additionally, the control system may be further configured to accentuate the pause count indicator in response to the number of transection pauses changing. In some embodiments, to communicate indicia of the number of transection pauses may include to generate an audible feedback and/or a haptic feedback indicative of the number of transection pauses. Additionally, in some embodiments, to communicate indicia of the number of transection pauses may include to display visual feedback on a graphical user interface. The visual feedback may include indicia of the number and location of each transection pause relative to each other.

In some embodiments, displaying visual feedback may include displaying a transection pause indicator array, wherein the transection pause indicator array includes a plurality of indicators arranged in positions relative to each other and wherein activation of an indicator of the plurality of indicators is indicative of a transection pause at a location indicated by the corresponding indicator. Additionally, displaying the visual feedback may include displaying an expected firing force profile graph having an ordinate axis of transection force and an abscissa axis of position of a knife of the end effector, and wherein the expected firing force profile graph includes indicia of the number and location of each transection pause. Furthermore, in some embodiments, the control system may be further configured to control the surgical stapler to perform the firing phase of the end effector, wherein to perform the firing phase includes to pause transection of the tissue by the end effector based on the number of transection pauses.

### BRIEF DESCRIPTION OF THE DRAWINGS

The detailed description particularly refers to the following figures, in which:
FIG. 1 is a perspective view of an embodiment of a surgical stapler configured for use with a robotic surgical system;
FIG. 2 is a perspective view of an embodiment of an end effector of the surgical stapler of FIG. 1;
FIG. 3 is an exploded perspective view of and embodiment of a distal end of the surgical stapler of FIG. 1, including the end effector of FIG. 2;
FIG. 4 is a perspective view of an embodiment of an I-beam of the end effector of FIG. 2, which includes a knife;
FIG. 5 is a cross-sectional view of the end effector of FIG. 1 in a closed state and showing the I-beam, of FIG. 4 received in corresponding channels of the end effector of FIG. 2 with the knife of the I-beam extending between the corresponding channels;
FIG. 6 is a perspective view of an embodiment of a stapler cartridge that may be inserted into the end effector of FIG. 2;
FIG. 7 is an exploded, perspective view of the stapler cartridge of FIG. 6;
FIG. 8 is a simplified block diagram of an embodiment of a robotic surgical system including a control console and a robotic manipulator, with which the surgical stapler of FIG. 1 may be used;
FIG. 9 is a diagrammatic view of an embodiment of a tool interface located on a bottom side of a drive housing of the surgical stapler of FIG. 1 being coupled to an arm interface located on a robotic arm of the robotic manipulator of the robotic surgical system of FIG. 8;
FIG. 10 is a perspective view of an embodiment of a hand-held surgical stapler including the end effector of FIG. 2;
FIG. 11 is a simplified block diagram of an embodiment of a control system that may be incorporated in the robotic manipulator and/or the control console of the robotic surgical system of FIG. 8;
FIGS. 12A-12C are a simplified flow diagram of a method for determining and scheduling transection pauses for a firing phase of the surgical stapler of FIG. 1, which may be executed by the robotic manipulator and/or the control console of the robotic surgical system of FIG. 8;
FIG. 13 is a simplified graph of work performed by the end effector of the surgical stapler during a compression phase of the surgical stapler of FIG. 1;
FIG. 14 is a simplified graph of a firing force profile including indicia of a number and location of transection pauses scheduled during the firing phase of the surgical stapler of FIG. 1;
FIG. 15 is simplified graph of an expected transection profile including indica of a number and location of scheduled transection pauses that may be displayed to a surgeon on the control console of the robotic surgical system of FIG. 8;
FIG. 16 is user interface including a pause count indicator indicative of the number of determined transection pauses that may be displayed to a surgeon on the control console of the robotic surgical system of FIG. 8;
FIG. 17 is another embodiment of a user interface including a pause indicator indicative of the number of determine transection pauses that may be displayed to a surgeon on a local display of the hand-held surgical stapler of FIG. 10;
FIG. 18 is another embodiment of the pause count indicator of the user interface of FIGS. 16 or 17 including an accentuation indicating that the pause count indicator has changed; and
FIG. 19 is a set of pause indicator arrays, each including indicia of a different number and location of determined transection pauses.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment," "an embodiment,""an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

Portions of the disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

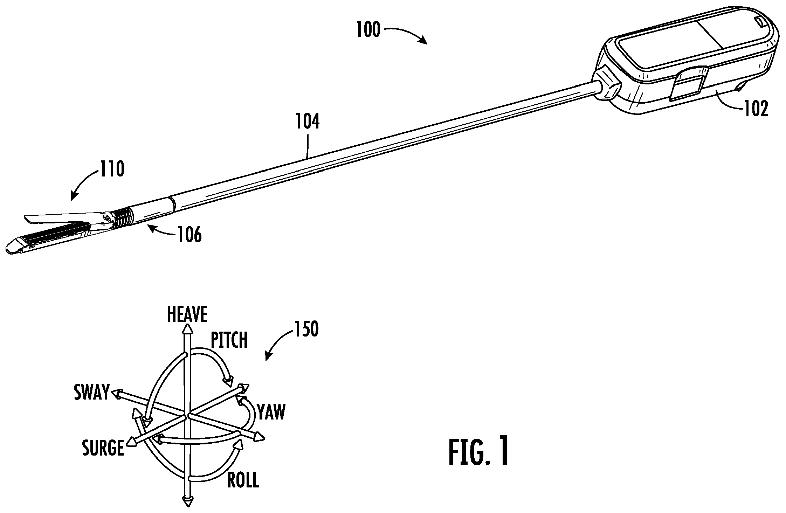

Referring now to FIG. 1, in an illustrative embodiment, a surgical stapler or "endocutter" 100 is configured for use in minimally invasive surgery (MIS) procedures including, but not limited to, endoscopic and laparoscopic procedures. The surgical stapler 100 is configured to contemporaneously transect and staple tissue during the performance of such surgical procedures. Although the concepts presented below are described in regard to a surgical stapler, it should be appreciated that the presented concepts may be applicable to other types of surgical instruments configured to perform different surgical functions including, but not limited to, surgical scissors, tissue graspers, energy-based surgical instruments, clip appliers, needle drivers, and/or other surgical instruments.

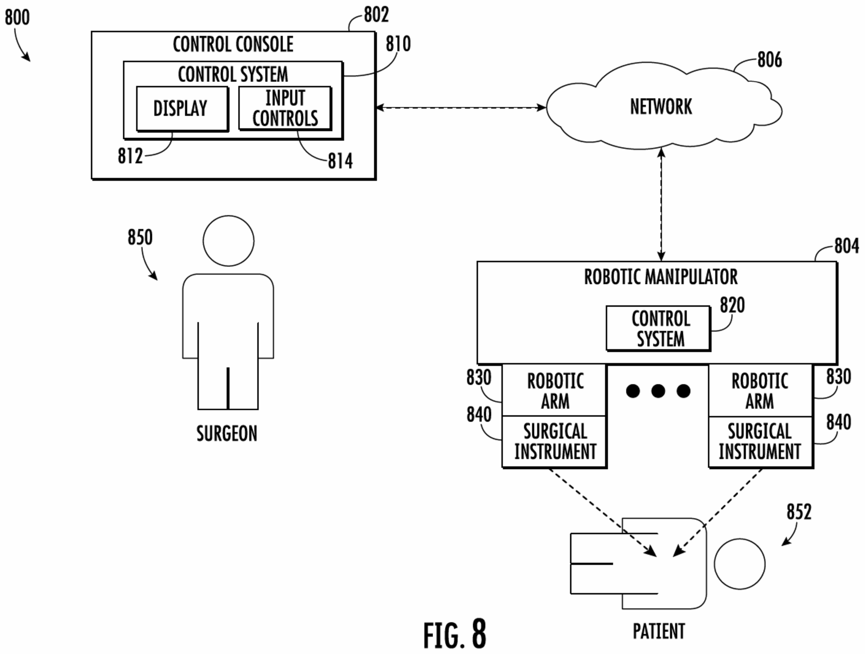

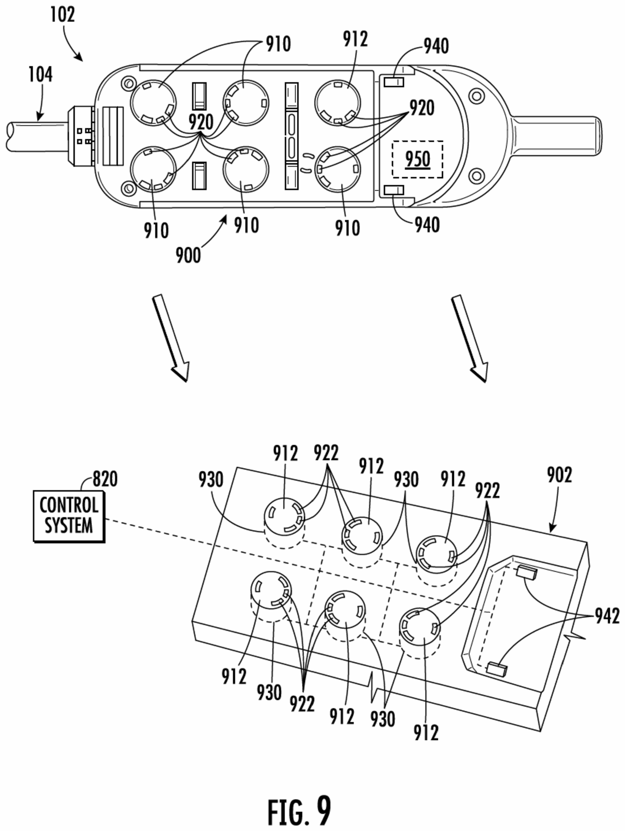

The illustrative surgical stapler 100 includes a drive housing 102, an elongated shaft 104 that extends distally away from the drive housing 102, and an end effector 110 located at a distal end of the elongated shaft 104 opposite the drive housing 102. As discussed in more detail below, the surgical stapler 100 is embodied as a robotic surgical stapler and is configured to be used with a corresponding robotic surgical system. As such, the drive housing 102 includes coupling features that that facilitate the mounting of the drive housing 102 to a robotic arm of a robotic manipulator of the robotic surgery system as discussed below in regard to FIGS. 8 and 9. The mounting of the drive housing 102 to the robotic arm allows the robotic manipulator to control various functions of the surgical stapler 100, including the movement and "firing" of the end effector 110, as discussed in more detail below.

The elongated shaft 104 is sized and configured for use in minimally invasive surgery procedures. For example, the elongated shaft 104 has a length and diameter that is sized to allow the elongated shaft 104 to be inserted into a trocar or similar surgical tube to allow positioning of the end effector 110 into the patient's anatomical environment (e.g., the patient's abdominal cavity). Illustratively, the distal end of the elongated shaft 104 is coupled to the end effector 110 via an articulable joint 106, which allows the end effector 110 to be moved to different orientations and/or positions relative to the elongated shaft 104.

In the illustrative embodiment, the articulable joint 106 provides six degrees of freedom to the end effector 110. For example, as indicated by coordinate frame 150, the degrees of freedom of the end effector 110 may include three translational degrees (i.e., surge, heave, and sway) and three rotational degrees (i.e., roll, pitch, and yaw). The "surge" degree of freedom refers to forward and backward translational movement of the end effector 110 relative to the elongated shaft 104, the "heave" degree of freedom refers to upward and downward translational movement of the end effector 110 relative to the elongated shaft 104, and the "sway" degree of freedom refers to left and right translational movement of the end effector 110 relative to the elongated shaft 104. The "roll" degree of freedom refers to rotation of the end effector 110 along a longitudinal axis defined by the end effector 110, the "pitch" degree of freedom refers to upward and downward tilting of the end effector 110 relative to the elongated shaft 104, and the "yaw" degree of freedom refers to leftward or rightward turning of the distal end of the end effector 110 relative to the elongated shaft 104.

The elongated shaft 104 also houses a portion of an actuation system (not shown) to control the movement and activation (e.g., the "firing") of the end effector 110. The actuation system may include various articulation cables, push rods, firing rods, and/or other devices, which extend through the elongated shaft 104 from mechanisms located in the drive housing 102 to components of the articulable joint 106 and/or the end effector 110. In this way, the drive housing 102 is configured to control the movement and activation (i.e., the "firing") of the end effector 110.

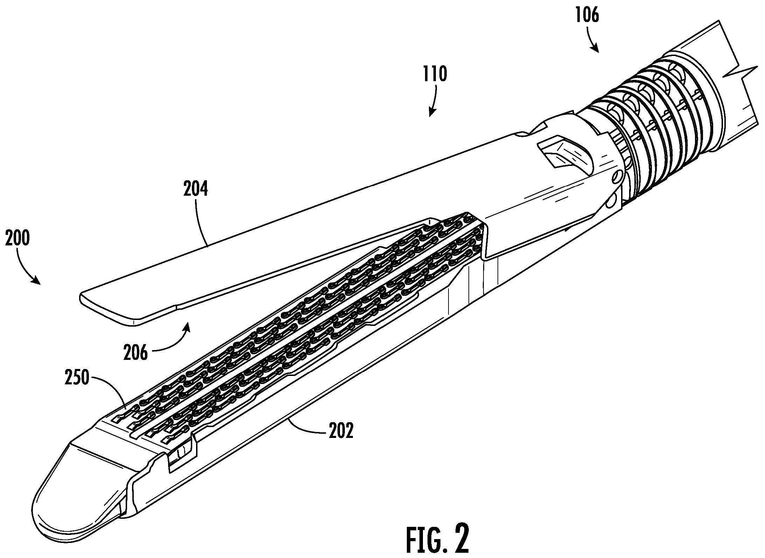

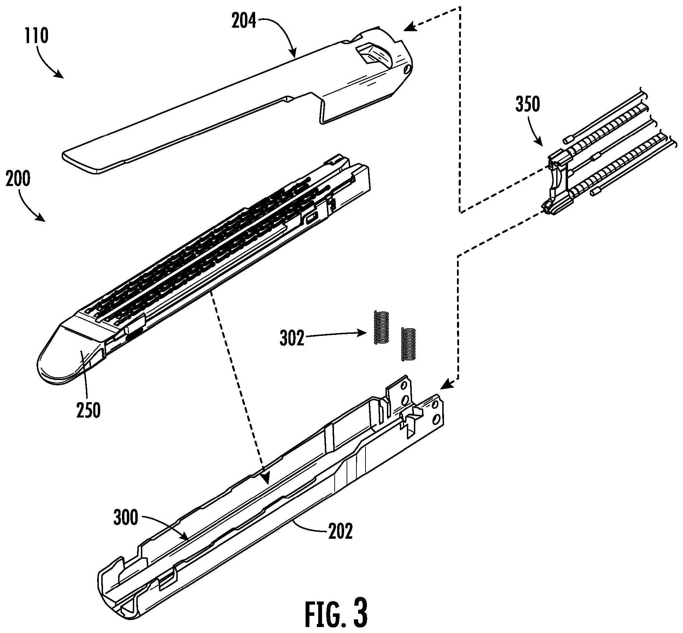

Referring now to FIG. 2, the end effector 110 includes a jaw assembly 200, which illustratively includes a pair of jaws 202, 204 that oppose each other. The jaw 202 is illustratively embodied as a "cartridge" jaw and includes a channel 300 (see FIG. 3) configured to receive a staple cartridge 250. The jaw 204 is illustratively embodied as an "anvil" jaw and includes a bottom surface 206 having forming pockets configured to deform staples ejected from the staple cartridge 250.

The jaw assembly 200 is movable between an open state in which the anvil jaw 204 is positioned away from the cartridge jaw 202 and a closed state in which the anvil jaw 204 is positioned near or otherwise contacts the cartridge jaw 202. Illustratively, the anvil jaw 204 is configured to move toward and away from the cartridge jaw 202. However, in other embodiments, the cartridge jaw 202 may be configured to move relative to the anvil jaw 204 or both jaws 202, 204 may be configured to move toward or away from each other. Additionally, it should be appreciated that the open state may correspond to a degree of openness that is less than a fully opened position of the jaw assembly 200 and the closed state may correspond to a degree of closeness that is less than a fully closed position. That is, the closed state may, for example correspond to a minimal distance between the distal ends of the cartridge jaw 202 and the anvil jaw 204 and the open state may correspond to a maximum distance between the distal ends of the jaws 202, 204. However, in other embodiments, the open state may correspond to a fully opened position of the jaw assembly 200 and the closed state may correspond to a fully closed position of the jaw assembly 200.

As shown in FIG. 3, the jaw assembly 200 is biased in the open state by a pair of springs 302. That is, the springs 302 bias the anvil jaw 204 away from the cartridge jaw 202. However, actuation of the jaw assembly 200 overcomes the biasing force of the springs 302 to move the jaw assembly 200 from the open state to the closed state. When in the closed state, the end effector 110 can be "fired" to effect the cutting and stapling of tissue held within the jaw assembly 200

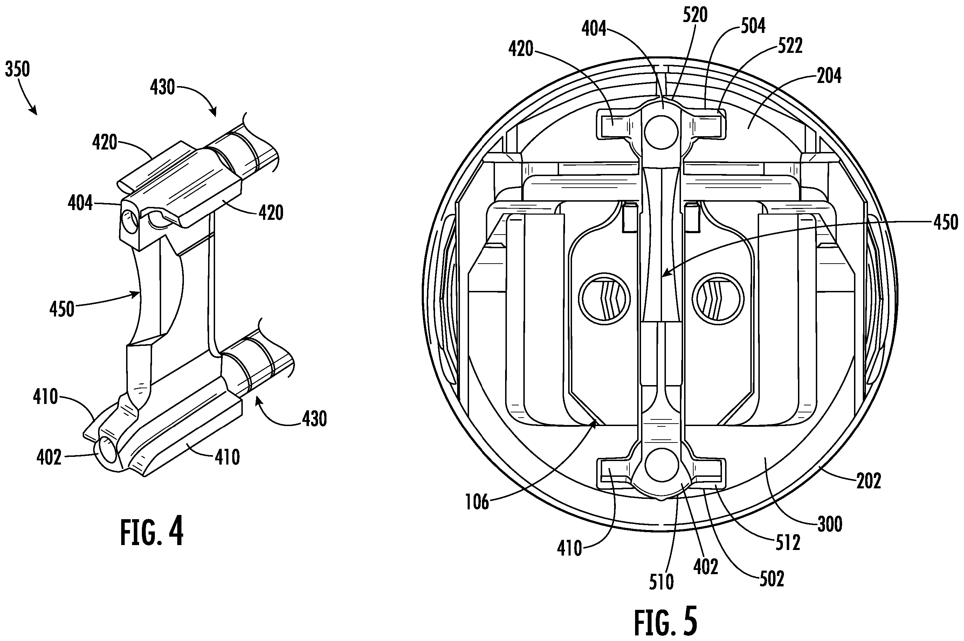

The end effector 110 also includes an I-beam 350, which is configured to translate forward during the firing of the end effector 110 from the proximal end of the jaws 202 toward the distal end of the jaws 202, 204. To do so, portions of the I-beam 350 are received in corresponding channels of the cartridge jaw 202 and the anvil jaw 204 as discussed in more detail below in regard to FIGS. 4 and 5. When the end effector 110 is fired, the I-beam 350 translates forward within the channels of the jaws 202, 204. The forward movement of the I-beam 350 may cause the anvil jaw 204 to move further downward toward or against the cartridge jaw 202 and clamp the jaw assembly in the closed state.

Referring now to FIGS. 4 and 5, the I-beam 350 includes a lower base 402, an upper base 404, and a knife 450 located between the upper and lower bases 402, 404. A pair of lower wings 410 extend laterally outward from the lower base 402. Similarly, a pair of upper wings 420 extend laterally outward from the upper base 404. A push rod 430 is coupled to each of the bases 402, 404 and to control mechanisms located in the drive housing 102. The control mechanisms of the drive housing 102 control movement of the push rods 430, which moves the I-beam 350 forward and backward within the jaw assembly 200.

When the end effector 110 is fired, the I-beam 350 moves within a lower I-beam channel 502 of the cartridge jaw 202 and an upper I-beam channel 504 of the anvil jaw 204 as shown in FIG. 5. The I-beam channel 502 of the cartridge jaw 202 includes a base channel 510 and a pair of wing channels 512, which are open to and in fluid communication with the base channel 510 and extend laterally outward therefrom. The lower base 402 of the I-beam 350 is received in and moves within the base channel 510 of the I-beam channel 502. Similarly, each lower wing 410 is received in and moves within a corresponding one of the wing channels 512. The I-beam channel 504 of the anvil jaw 204 also includes a base channel 520 and a pair of wing channels 522, which are open to and in fluid communication with the base channel 520 and extend laterally outward therefrom. The upper base 404 of the I-beam d 350 is received in and moves within the base channel 520 of the I-beam channel 502. Similarly, each upper wing 420 is received in and moves within a corresponding one of the wing channels 522.

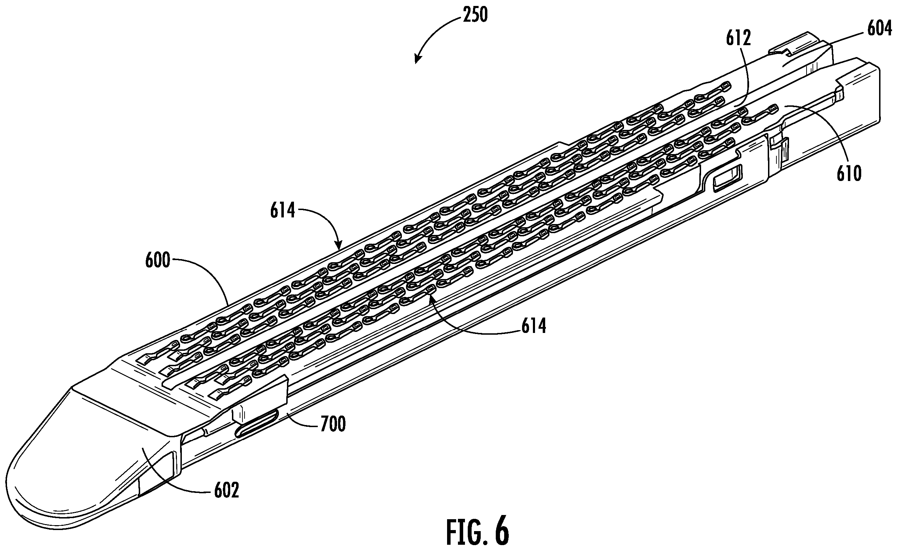

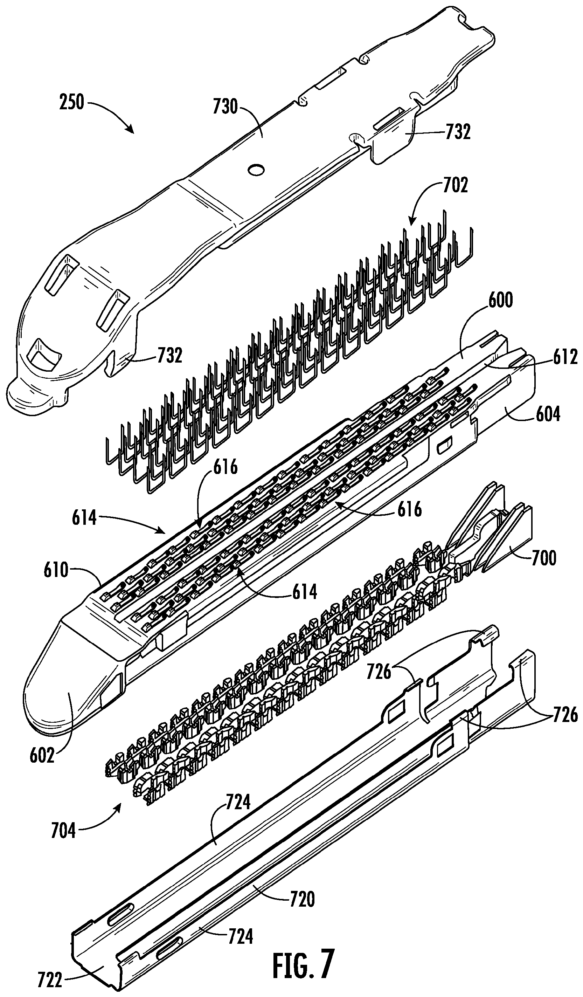

Referring now to FIGS. 6 and 7, the illustrative staple cartridge 250 includes a cartridge body 600 having a distal end 602 and a proximal end 604. The cartridge body 600 also includes a deck 610 that extends from the proximal end 604 to the distal end 602. The deck 610 includes a longitudinal knife slot 612 that is located centrally on the deck 610 and extends from the distal end 602 toward the proximal end 604 of the cartridge body 600. During a firing stroke of the surgical stapler 100 (i.e., when the end effector is "fired"), the I-beam 350 transversed along the longitudinal knife slot 612 with the knife 450 of the I-beam 350 protruding upwardly from the longitudinal knife slot 612 to facilitate the transection of tissue captured within in the end effector 110 and held between the staple cartridge 250 and the anvil jaw 204.

A set of staple cavities 614 are defined in the deck 610 on either side of the longitudinal knife slot 612. A staple 702 is positioned in each of the staple cavities 614 and is supported by a corresponding staple driver 704. The staple cartridge 250 also includes a staple sled 700, which is initially located toward the proximal end 604 of the cartridge body 600 and is pushed toward the distal end 602 by the I-beam 350 when the end effector 110 is fired. The staple sled 700 lifts each staple driver 704 when the sled 700 comes into contact with the corresponding staple driver 704, which ejects the staple 702 associated with the corresponding staple driver 704 from the corresponding staple cavity 614. In the illustrative embodiment, the deck 610 includes staple guides or projections 616 that project upwardly from the deck 610 around each staple cavity 614. The staple guides 616 are configured to guide or control the legs of the staples 702 as the staples 702 are being ejected from the staple cavities 614. Illustratively, the staple guides 616 are located around the distal and proximal ends of each staple cavity 614, but may completely surround each staple cavity 614 or be located on only one end of each staple cavity 614 in other embodiments. The staple guides 616 may also form a set of "teeth" and be configured to grasp tissue held within the jaw assembly 200 to restrict movement of the tissue. In other embodiments, the deck 610 of the cartridge body 600 maybe devoid of any staple guides 616.

The staple cartridge 250 also includes a pan 720, which is attached to the cartridge body 600 and is configured to retain the staple drivers 704 and associated staples 702 within the cartridge body 600. Illustratively, the pan 720 is formed from a metallic material and includes a floor 722 and a pair of sidewalls 724 that extend upwardly from the floor 722 and wrap onto the sides of the cartridge body 600. The pan 720 includes a set of attachment tabs 726 that are configured to secure the pan 720 to the cartridge body 600.

Prior to use, the staple cartridge 250 includes a cover 730 attached to the cartridge body 600. The cover 730 is configured to cover the deck 610 of the staple cartridge 250 and includes a set of attachment tabs 732 configured to secure the cover 730 to the cartridge body 600. To prepare the staple cartridge 250 for use in the end effector 110, the cover 730 is removed from the cartridge body 600.

Although not illustrated in FIGS. 6 and 7, the staple cartridge 250 may also includes one or more electronic circuits or devices configured to perform one or more associated functions. For example, such electronic circuitry may include processors, digital storage devices, communication circuitry, sensor circuitry, and/or other electrical components.

Referring now to FIG. 8, as discussed above, the illustrative surgical stapler 100 is configured for use with a corresponding robotic surgical system 800. The robotic surgical system 800 includes a control console 802 and a robotic manipulator 804, which communicate with each other over a communication network 806. Although only a single control console 802 and a single robotic manipulator 804 is shown in FIG. 8, it should be appreciated that the robotic surgical system 800 may include additional control consoles 802 and/or robotic manipulators 804 in other embodiments.

The control console 802 is usable by a surgeon 850 to control the operation of the robotic manipulator 804. To do so, the control console 802 includes a control system 810. Illustratively, the control system 810 includes a display 812 and one or more input controls 814. However, it should be appreciated that the control system 810 may include additional electrical components and devices, such as a processor, a memory, and a communication subsystem to enable communications of the components of the control system 810, which are not illustrated in FIG. 8 for clarity.

The display 812 may be embodied as any type of display device capable of generating images viewable by the surgeon 850. In use, the display 812 may display images related to the surgical procedure being performed via the robotic manipulator 804. The displayed images may be obtained from, for example, an endoscopic camera operated by the robotic manipulator 804. Additionally, the display 812 may display information, including data determined by the control console 802 and/or the robotic manipulator 804, related to the surgical procedure (e.g., positional data of the robotic manipulator 804).

The input controls 814 are usable by the surgeon 850 to control the functionality of the robotic manipulator 804. The input controls 814 may be embodied as any type of input device capable of receiving a corresponding input from the surgeon 850. For example, the input controls 814 may include physical controllers, such as joy sticks, hand-held actuator modules, exoskeletal gloves, and/or other input devices. The input controls 814 may also include input devices other than hand-controlled devices such as foot pedals, vison tracking modules, and/or the like. In many embodiments, the input controls 814 are movable in multiple degrees of freedom to control the positioning and operation of the robotic manipulator 804.

The robotic manipulator 804 also includes a control system 820 and one or more robotic arms 830 to which surgical instruments 840 (e.g., the surgical stapler 100) may be mounted. In some embodiments, the robotic manipulator 804 may be mounted to a transport cart, sometimes referred to as an "arm cart," that enables mobility of the robotic manipulator 804 and the associated robotic arms 830.

The robotic arms 830 may include various articulable linkages and associated motors, which are controllable by the control console 802 to move the corresponding robotic arm 830 and any associated surgical instrument 840 to a desired position. For example, by manipulating an input control 814 of the control console 802, the surgeon 850 may control the positioning of a corresponding robotic arm 830, as well as the functionality of the associated surgical instrument 840 (e.g., the firing of the surgical stapler 100).

The control system 820 may be embodied as any type of controller or control circuit capable of controlling the functionality of the robotic manipulator including, for example, the movement of the robotic arms 830 and the activation of the surgical instruments 840 based on control signals received from the control console 802. To do so, the control system 820 may include various electrical components, circuits, and/or devices, such as a processor, a memory, and a communication subsystem to enable communications of the components of the control system 820, which are not illustrated in FIG. 8 for clarity.

In use, the robotic manipulator 804 is positioned in close proximity to a patient 852 requiring surgery. The robotic manipulator 804 may be locked or mounted in place for the duration of the surgery. The surgeon 850 may then manipulate the input controls 814 to position one or more robotic arms 830 and associated surgical instruments 840 into a desired position. For example, the surgeon 850 may position a robotic arm 830 such that an associated surgical instrument 840 is inserted through a trocar or similar elongated passageway into the anatomical environment (e.g., the abdominal cavity of the patient 852). Once so positioned, some surgical instruments 840 (e.g., an endoscope) may be locked into position to avoid unintended repositioning.

The network 806 may be embodied as any type of wired and/or wireless network or set of communication links capable of facilitating communications between the control console 802 and the robotic manipulator 804. To do so, the network 806 enable such communications using any suitable data communication specification and/or protocol. As such, in some embodiments, the network 806 may include additional devices, such as additional computers, routers, stations, and/or switches, to facilitate such communications between the control console 802 and the robotic manipulator 804.

Referring now to FIG. 9, as discussed above, the surgical stapler 100 (and other surgical instruments 840) is configured to be mounted to a robotic arm 830 of the robotic manipulator 804. To facilitate such mounting, the drive housing 102 of the surgical stapler 100 includes a tool interface 900 located on a bottom side of the drive housing 102. The tool interface 900 is configured to mate with an arm interface 902 of the corresponding robotic arm 830 to couple the surgical stapler 100 to the robotic arm 830. The coupling of the surgical stapler 100 to the robotic arm 830 may be further facilitated via various mechanical, magnetic, and/or electrical features. In some embodiments, a sterile barrier may also be used between the surgical stapler 100 and the robotic arm 830. It should be appreciated that mounting of the surgical stapler 100 to the robotic arm places the surgical stapler 100 into communication and under the control of the control system 820 of the robotic manipulator 804, which is controlled by the control console 802 as discussed above.

The tool interface 900 includes a set of input pucks 910, which are manipulable to control functions of the surgical stapler 100 such as the positioning and firing of the end effector 110. Each input puck 910 is configured to mate with a corresponding puck driver 912 of the arm interface 902. To do so, each input puck 910 includes mating features 920 that are configured to mate with mating features 922 of the corresponding puck driver 912. In the illustrative embodiment, the mating features 920 of the input pucks 910 are embodied as tabs or protrusions that extend upwardly from the corresponding input puck 910, and the mating features 922 of the puck drivers 912 are embodied as recesses configured to receive the tabs/protrusions 920 of the input pucks 910. In other embodiments, however, the mating features 920 of the input pucks 910 may be embodied as recesses and the mating features 922 of the puck drivers 912 may be embodied as tabs/protrusions.

The arm interface 902 includes an electric motor 930 operatively coupled to each puck driver 912. Each electric motor 930 is communicatively coupled to and controlled by the control system 820. Actuation of a given electric motor 930 causes actuation of the associated puck driver 912, which causes actuation of the corresponding input puck 910. For example, rotation of an electric motor 930 causes rotation of the associated puck driver 912, which causes rotation of the corresponding input puck 910. In this way, the positioning and activation (e.g., "firing") of the end effector 110 of the surgical stapler 100 may be controlled via actuation of the puck drivers 912 and associated input pucks 910.

The tool interface 900 also includes a set of electrical connectors 940, which are configured to mate with corresponding electrical connectors 942 of the arm interface 902. The electrical connectors 940, 942 provide electrical communication between the surgical stapler 100 and the robotic manipulator 804. However, in other embodiments, the surgical stapler 100 and the robotic manipulator 804 may be configured to communicate in other ways, such as via wireless communications. In some embodiments, the tool interface 900 may also include a control system 950. In such embodiments, the control system 950 may be configured to control and/or monitor various operations the surgical stapler 100. The control system 950 may include various electrical components, circuits, and/or devices, such as a processor, a memory, and a communication subsystem to enable communications of the components of the control system 950, which are not illustrated in FIG. 9 for clarity.

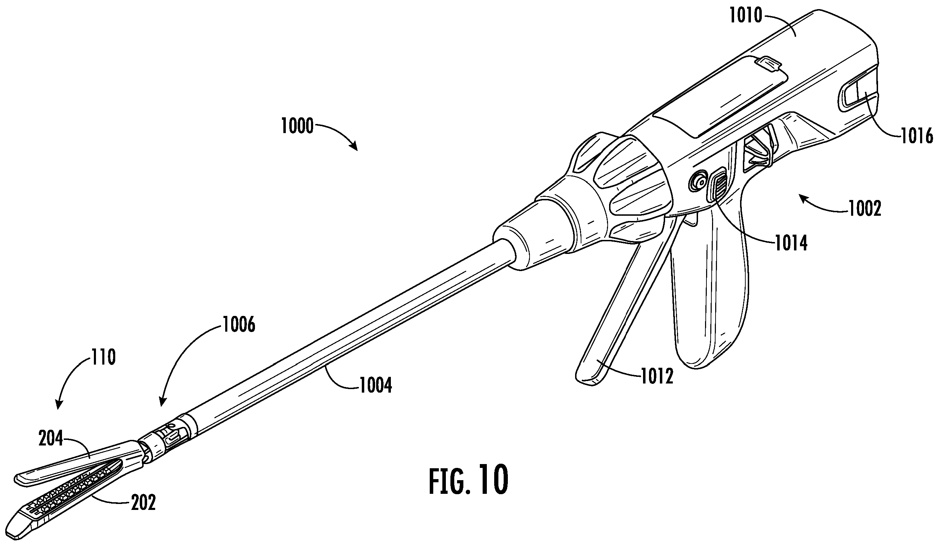

Although the illustrative surgical stapler 100 shown and described above in regard to FIGS. 1-9 is configured for use in a robotic surgical system, the features and concepts of the surgical stapler 100 described above and below may be applicable to hand-held surgical staplers and devices. For example, as shown in FIG. 10, the end effector 110 may be incorporated into a hand-held surgical stapler 1000. The surgical stapler 1000 includes a handle 1002 and an elongated shaft 1004 extending from the handle 1002. The distal end of the elongated shaft 1004 is coupled to the end effector 110 via an articulable joint 1006, which may be substantially similar to the articulable joint 106 described above.

The handle 1002 includes a handle housing 1010 and a trigger assembly 1012 movable connected to the handle housing 1010. The trigger assembly is operable by a surgeon to move the jaw assembly 200 of the end effector 110 from the open state to the closed state as discussed above. The handle 1002 also includes one or more activation triggers 1014 to, for example, initiate the firing of the end effector 110 when in the closed state. A replaceable and/or rechargeable battery pack 1016 is coupled to an end of the handle housing 1010 and provide power to the electrical components located within the handle housing 1010.

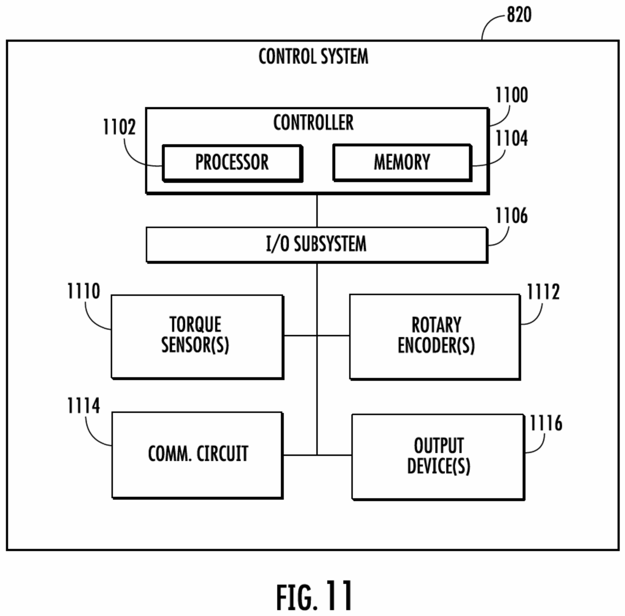

Referring now to FIG. 11 and as discussed above, an illustrative control system 820 is configured to control the functionality of the robotic manipulator 804 and surgical instruments 840 (e.g., the surgical stapler 100) operably coupled to a robotic arm 380 of the robotic manipulator 804. In use, as described in more detail below, the control system 820 is configured to determine an amount of work performed by the jaw assembly 200 of the end effector 110 of the surgical stapler 100 to compress tissue captured within the jaw assembly 200 and determine a number of transection pauses to be scheduled during the firing phase of the end effector 110 based on the determined amount of work. In doing so, the control system 820 may determine both the number and relative temporal positioning of the transection pauses during the firing phase. The control system 820 communicates the number and, in some embodiments, the relative positioning of the determined transection pauses to the operator of the robotic surgical system 800 (e.g., the surgeon 850 operating the control console 802). It should be appreciated that by communicating the planned number and relative location of the transection pauses, the surgeon's 850 performance of the surgery using the robotic surgical system 800 may be improved. For example, the surgeon the disclosed technologies may allow surgeons to develop a mental model of how cartridge type, tissue type, and the number and location of transection pauses affect patient outcomes. Additionally, by providing the surgeon with an indication of the transection pause plan (i.e., the number and location of pauses), the surgeon will be better prepared for experiencing those pauses, which may lead to better surgical performance.

The control system 820 may be embodied as any type of controller or control circuit capable of performing the functions described above and further below. Although the control system 820 is illustrated and described above in regard to FIG. 8 as being included in the robotic manipulator 804, it should be appreciated that, in some embodiments, the control system 820 and the associated functionality may be distributed across multiple components of the robotic surgical system 800, such as the control console 802, the robotic manipulator 804, one or more robotic arms 830, and/or a surgical instrument 840 (e.g., the surgical stapler 100). In such embodiments, the control system 820 may be embodied as a "logical" control system or computer.

In the illustrative embodiment, the control system 820 includes a controller 1100, an I/O subsystem 1102, one or more torque sensors 1110, one or more rotary encoders 1112, a communication circuit 1114, and one or more output devices 1116. It should be appreciated, however, that the control system 820 may include other or additional components such as those commonly found in an embedded computer or computer system. Additionally, in some embodiments, one or more of the illustrative components of the control system 820 may be incorporated in, otherwise form a portion of, another component.

The controller 1100 may be embodied as any type of device or collection of devices capable of performing various compute and/or control functions, as described below. In some embodiments, the controller 1100 may be embodied as a single device such as an integrated circuit, an embedded system, a field-programmable-array (FPGA), a system-on-a-chip (SOC), or other integrated system or device. Additionally, in some embodiments, the controller 1100 includes or is embodied as a processor 1102 and memory 1104. The processor 1102 may be embodied as any type of processor capable of performing the functions described herein. For example, the processor 1102 may be embodied as a single or multi-core processor(s), digital signal processor, microcontroller, or other processor or processing/controlling circuit. Similarly, the memory 1104 may be embodied as any type of volatile and/or non-volatile memory and/or data storage capable of storing data generated by or otherwise obtained by the control system 820. In operation, for example, the memory 1104 may store various data and software used during operation of the control system 820 such as operating systems, applications, programs, libraries, and drivers.

The controller 1100 is communicatively coupled to other components of the control system 820 via the I/O subsystem 1106, which may be embodied as circuitry and/or components to facilitate input/output operations with the controller 1100 (e.g., with the processor 1102 and/or memory 1104) and other components of the control system 820. For example, the I/O subsystem 1106 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations.

The torque sensor(s) 1110 may be embodied as any type of sensor capable of producing sensor data indicative of the torque of one or more electric motors 930 of an arm interface 902 of a robotic arm 830 of the robotic manipulator 804. Specifically, the torque sensor 1110 is configured to generate sensor data indicative of the present torque of the one or more electric motors 930 configured to control actuation of the jaw assembly 200 of the end effector 110 of the surgical stapler 100 coupled to the respective robotic arm 830. In this way, the torque sensor(s) 1110 is capable of sensing the amount of torque required by the corresponding electric motor(s) 930 to compress tissue captured within the jaw assembly 200. Alternatively, in other embodiments, other types of sensors configured to produce sensor data indicative of the amount of work required to compress the tissue captured within the jaw assembly 200 may be used. For example, the control system 820 may include sensors or circuits configured to measure motor angle, motor current, drivetrain torque and/or angle, drivetrain force and/or position, strain sensors, etc. Such sensors may be located within the drive housing 102, the elongated shaft 104, and/or the jaw assembly 200.

Similarly, the rotary encoder(s) 1112 may be embodied as any type of sensor capable of producing sensor data indicative of a present rotational angle of the one or more electric motors 930. Specifically, the rotary encoder is configured to generate sensor data indicative of rotational angle of the one or more electric motors 930 configured to control actuation of the jaw assembly 200 of the end effector 110 of the surgical stapler 100 coupled to the respective robotic arm 830. In this way, the rotary encoder(s) 1112 is capable of sensing the amount of rotation required by the corresponding electric motor(s) 930 to compress tissue captured within the jaw assembly 200.

The communication circuit 1114 of the control system 820 may be embodied as any type of communication circuit, device, or collection thereof, capable of enabling communications between the robotic manipulator 804 and the control console 802 over the network 806. To do so, the communication circuit 1114 may utilize any suitable communication protocol including, but not limited to, Ethernet, Wi-Fi (e.g., communications based on the Institute of Electrical and Electronics Engineers (IEEE) 802.11 family), a proprietary protocol, and/or other communication protocols.

The control system 820 also includes one or more output device(s) 1116. Each output device 1116 may be embodied as any type of device or collection of devices capable of generating an output detectable by an operator of the robotic surgical system 800 (e.g., the surgeon 850 operating the control console 802). For example, in some embodiments, the output device(s) 1116 is embodied as, or otherwise includes, a visual output device capable of generating a visual output, such as a display screen (e.g., display 812 of the control system 810 of the control console 802), a light (e.g., a light located on the control console 802 and/or the robotic manipulator 804), and/or other visually-detectable output.

Additionally or alternatively, the output device(s) 1116 is embodied as, or otherwise includes, an audible output device capable of generating an audible output, such as speaker or horn (e.g., a speaker located on the control console 802 and/or on the robotic manipulator 804). Further, in some embodiments, the output device(s) 1116 is embodied as, or otherwise includes, a haptic or tactile output device capable of generating a "touch" output. For example, the output devices 1116 may include a haptic output device incorporated in the input controls 814 of the control console 802 to provide an amount of force-feedback to the surgeon 850. As such, it should be appreciated that the output devices 1116 may include output devices located on the control console 802, the robotic manipulator 804, one or more robotic arms 830, and/or the surgical instrument 840.

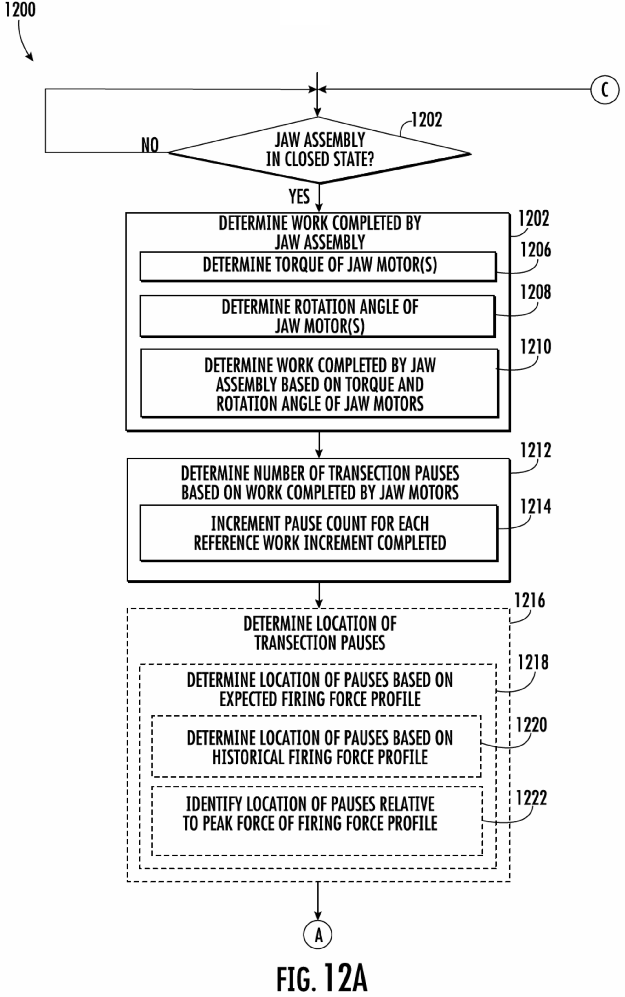

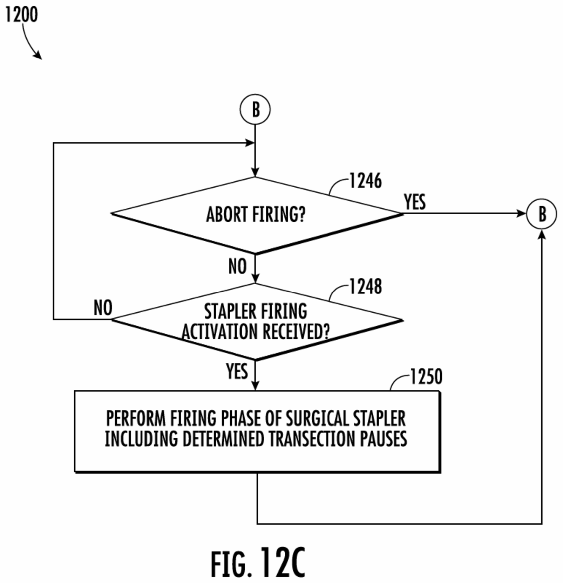

As discussed above, the illustrative control system 820 is configured to control operation of a surgical stapler 840 (or other surgical instrument) coupled to a robotic arm 830 of the robotic manipulator 804 by determining a number of transection pauses to be incorporated into a firing phase of the end effector 110 of the surgical stapler 100. To do so, the control system 820 may execute a method 1200 as shown in FIGS. 12A-12C.

The method 1200 begins with block 1202 in which the control system 820 determines whether the jaw assembly 200 of the end effector 110 of the associated surgical stapler 100 is in a closed state. In the illustrative embodiment, the closed state determined in block 1202 corresponds to a closed state of the jaw assembly 200 in which tissue captured in the jaw assembly 200 is being compressed, after an initial grasping phase, and the jaw assembly 200 is in a position in which the firing phase of the end effector 110 may be performed to transect and staple the compressed tissue.

If the control system 820 determines that the jaw assembly 200 is in the suitable closed state, the method 1200 advances to block 1204. In block 1204, the control system 820 determines a total amount of work performed by the jaw assembly 200 of the end effector 110 of the associated surgical stapler 100 to compress the tissue grasped within the jaw assembly 200. To do so, the control system 820 may utilize any suitable methodology to determine the work performed by the jaw assembly 200.

In the illustrative embodiment, the control system 820 is configured to determine the work performed based on the amount of torque and the rotational angle of the electric motor(s) 930 of the arm interface 902 configured to control the jaw assembly 200, via the tool interface 900 of the surgical stapler 100, to compress the tissue. As such, in block 1206, the control system 820 determines the present amount of torque of the relevant electric motor(s) 930 required to compress the tissue based on the sensor data received from the torque sensor(s) 1110. Similarly, in block 1208, the control system determines the present rotational angle of the relevant electric motor(s) 930 required to compress the tissue based on the sensor data received from the rotary encoder(s) 1112.

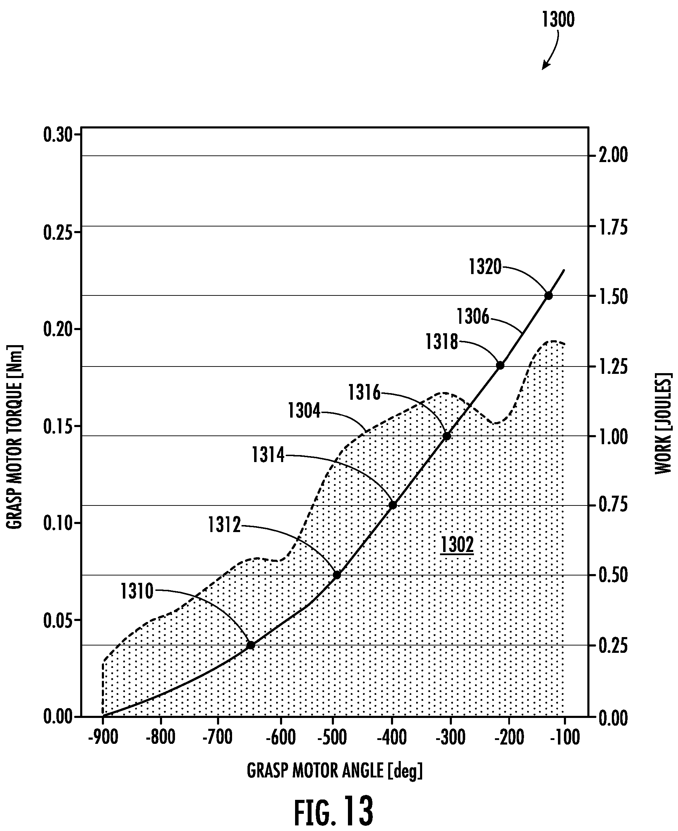

Subsequently, in block 1210, the control system 820 determines the work completed by the jaw assembly 200 to compress the tissue based on the amount of torque determined in block 1206 and the rotation angle determined in block 1208 of the electric motor(s) 930 configured to control the jaw assembly, as discussed above. To do so, the control system 820 is configured to prepare and/or analyze a graph 1300 of motor torque-to-motor angle of the relevant electric motor(s) 930 as shown in FIG. 13. The amount of work performed by the jaw assembly 200 is represented as an amount of area 1302 occurring under an upper boundary 1304 of the measured motor torque to measured motor angle. By integrating that upper boundary 1304, the control system 820 can determine the total amount of work 1306 performed by the jaw assembly 200 to compress the tissue, which is represented in graph 1300 as a line plot.

As discussed in more detail below, the control system 820 may also determine a number of reference or predetermined work increments completed (or partially completed) within the total amount of work completed. For example, in the illustrative embodiment of FIG. 13, each individual reference work increment is set to 0.25 Joules, and the illustrative total work completed 1306 is divided into six reference work increments 1310, 1312, 1314, 1316, 1318, 1320 as indicated in graph 1300. Each of the reference work increments 1310, 1312, 1314, 1316, 1318, 1320 of the total work 1306 performed by the jaw assembly 200 is correlated to a transection pause as described further below.

Referring back to FIG. 12A, after the control system 820 has determined the amount of work performed by the jaw assembly 200 to compress the tissue in block 1202, the method 1200 advances to block 1212. In block 1212, the control system 820 determines a number of transection pauses to be scheduled in the firing phrase of the end effector 110 based on the total amount of work determined in block 1204. To do so, in block 1214, the control system 820 increments a pause count, which is indicative of the number of transection pauses to be scheduled for each reference work increment completed (or partially completed) within the total amount of work completed by the jaw assembly 200 to compress the tissue. In the illustrative embodiment, as discussed above, each reference work increment is equal to 0.25 Joules, but may be set to a different amount of work in other embodiments. As such, for each reference work increment amount performed by the jaw assembly 200, the pause count is incremented. It should be appreciated that such adjustment of the pause count may occur while the jaw assembly 200 is in the process of compressing the tissue or may be determined after compression of the tissue by the jaw assembly is completed. Additionally, it should be appreciated that each determined transection pause is indicative of a period of time during the firing phase of the end effector 110 of the surgical stapler 100 during which the knife 450 of the I-beam 350 will be halted. That is, the knife 450 is not advanced during the corresponding transection pause and, as such, cutting of the tissue grasped by the jaw assembly 200 is momentarily paused.

In some embodiments, in block 1216, the control system 820 may also determine a location for each transection pause during the firing phase. To do so, in the illustrative embodiment in block 1218, the control system 820 may determine a scheduled temporal location for each transection pause based on an expected firing force profile of the firing phase of the end effector 110. As shown in block 1220, the expected firing force profile may be based on, for example, one or more historical firing force profiles of the end effector 110 (e.g., benchtop testing, pre-clinical labs, clinical labs, field data, etc.), which may be updated over time and retrieved by control system 820. In this way, the expected firing force profile may be embodied as a statically derived profile. Alternatively, the expected firing force profile may be embodied as a default firing force profile for the particular end effector 110 and/or staple cartridge 250 in use.

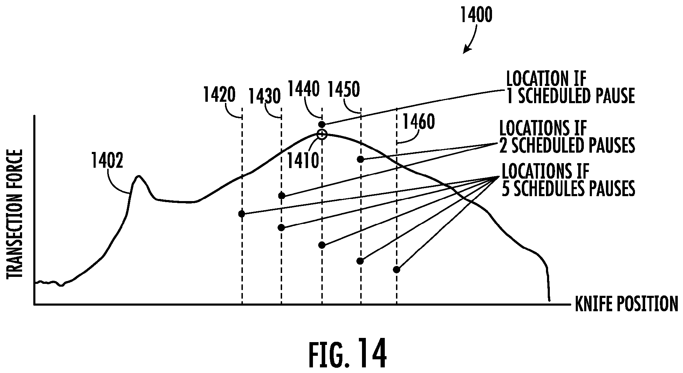

Regardless, based on the expected firing force profile, the control system 820 is configured to determine a location for each transection pause relative to a peak firing force of the end effector 110 in block 1222. For example, an expected firing force profile 1402 for an end effector 110 is illustrated in a graph 1400 in FIG. 14. The control system 820 determines a peak firing force 1410 (indicated as a circle in FIG. 14) of the firing force profile 1402 and schedules the determined number of transection pauses relative to the determined peak firing force. For example, if a single transection pause is to be scheduled, the control system 820 schedules the transection pause to correlate to the peak firing force 1410 as indicated by vertical line 1440. Alternatively, if two transection pauses are to be scheduled, the control system 820 evenly spaces the two transection pauses about the peak firing force 1410 as indicated by vertical lines 1430, 1450. If, however, five transection pauses are to be scheduled, the control system 820 evenly spaces the transection pauses about the peak firing force 1410 (including a transection pause occurring on the peak firing force 1410) as indicated by vertical lines 1420, 1430, 1440, 1450, 1460. As such, in the illustrative embedment, the control system 820 utilizes a "middle-out" scheduling algorithm, relative to the peak firing force 1410, to determine the location of each transection pause. It should be appreciated, however, that the control system 820 may use a different methodology to schedule the location of each determined transection pause in other embodiments. For example, the transection pauses may be scheduled relative to the beginning of the firing phrase (e.g., evenly spaced from the beginning of the firing phase of the end effector 110) in some embodiments.

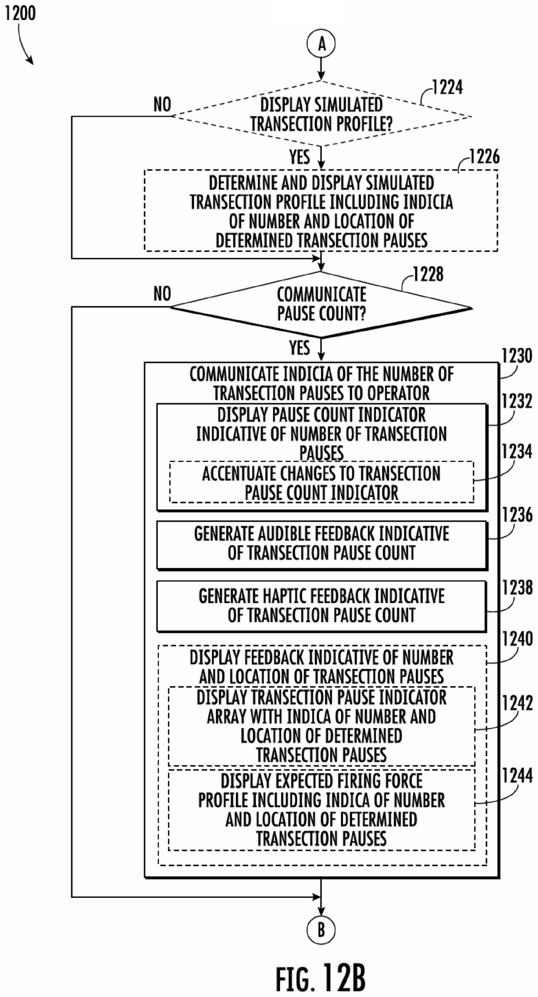

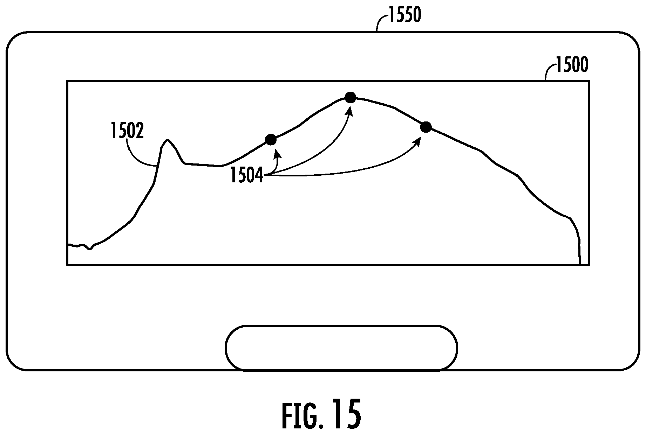

Referring back to FIG. 12A, after the control system 820 has determined the location for each transection pause in block 1216, the method 1200 advances to block 1224 of FIG. 12B. In block 1224, the control system 820 determines whether the operator (e.g., surgeon 850) of the robotic surgical system 800 desires to review a simulated transection profile. For example, the operator may provide an appropriate input to view the simulated transection profile prior to activating the firing phase of the end effector 110 of the surgical stapler 100. If so, the method 1200 advances to block 1226 in which the control system 820 determines and displays a simulated transection profile that includes indica of the number and relative location of each determined transection pause. For example, as shown in FIG. 15, the control system 820 may display a simulated transection profile 1500 on a graphical user interface 1550 displayed to the operator on the display 812 of the control console 802. The simulated transection profile 1500 includes a simulated firing force plot 1502 including indica 1504 of each transection pause (illustratively three pauses), which are placed on the firing force plot 1502 in appropriate locations to indicate the relative positioning of each transection pause. It should be appreciated that the simulated transection profile 1500 may provide a visual reference to the operator of the control console 802 of the scheduled transection pauses and resulting transection profile prior to the firing of the end effector 110.

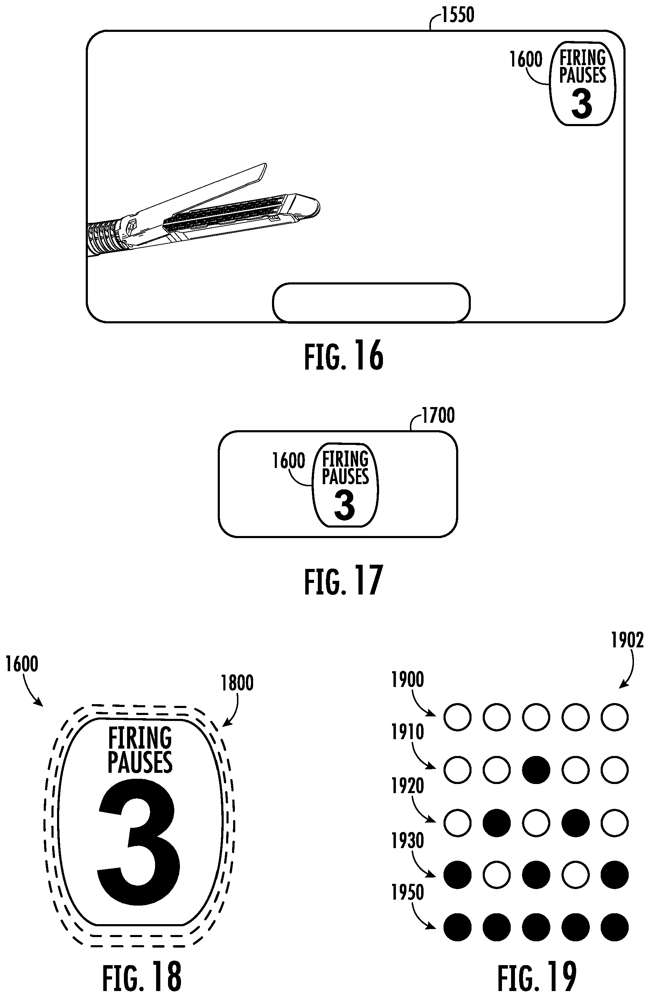

Referring back to FIG. 12B, after the control system 820 has determined and displayed the simulated transection profile or if no profile is to be displayed to the operator, the method 1200 advances to block 1228. In block 1228, the control system 820 determines whether to communicate the pause count (i.e., the number of determined transection pauses) to the operator of the robotic surgical system 800. If so, the method 1200 advances to block 1230 in which the control system 820 communicates indicia of the number of transection pauses, as determined in block 1212, to the operator. To do so, the control system 820 may utilize any one or more modalities to communicate the number of transection pauses. For example, in block 1232, the control system 820 may display a pause count indicator that is indicative of the number of transection pauses, which is based on the total amount of work performed by the jaw assembly 200 to compress the tissue grasped within the jaw assembly 200 as determined in block 1212 described above. As shown in FIG. 16, for example, the control system 820 may display a pause count indicator 1600 on the graphical user interface 1550, which is displayed to the operator (e.g., the surgeon 850) of the control console 802 via the display 812. The illustrative pause count indicator 1600 display the number of determined transection pauses and, in some embodiments, may be updated as that number increases (e.g., based on the present work performed by jaw assembly 200 to compress the tissue). In those embodiments in which the surgical stapler is embodied as a hand-held stapler 1000 as shown in FIG. 10, the control system 820 may be configured to display the pause count indicator 1600 on a local display 1700 of the hand-held stapler 1000 as shown in FIG. 17. The local display 1700 may be incorporated into the housing of the hand-held stapler 1000 to provide a visual indication of the transection pause count to the surgeon.

Referring back to FIG. 12B, in some embodiments in block 1234, the control system 820 may also be configured to accentuate such changes or updates to the pause count indicator. For example, as shown in FIG. 18, the control system 820 may be configured to display a border 1800 around the pause count indicator 1600, change the color of the border 1800 and/or the pause count indicator 1600, flash or provide movement to border 1800, or otherwise accentuate the pause count indicator 1600 each time the number of determined transection pauses increases or otherwise changes.

Referring again back to FIG. 12B, additionally or alternatively, the control system 820 may be configured to generate an audible feedback indicative of the transection pause count in block 1236. For example, such audible feedback may be embodied as a beep, buzz, or noise for each determined transection pause. Additionally or alternatively, in block 1238, the control system 820 may be configured to generate a haptic or tactile feedback indicative of or based on the transection pause count. For example, the control system 820 may provide a haptic feedback (e.g., a "shake" response) on one or more of the input controls 814 of the control console 802 indicative of or based on the transection pause count.

In embodiments in which the control system 820 has determined both the number and relative location of transection pauses, the control system 820 may display feedback to the operator of the robotic surgical system 800 indicative of the number and the relative location of the determined transection pauses in block 1240. For example, in block 1242 and as shown in FIG. 19, the control system 820 may display a transection pause indicator array 1900, which includes a number of indicators 1902. Each indicator 1902 correlates to a corresponding transection pause and the relative positioning of each indicator 1902 provides an indication of the relative positioning of the corresponding transection pause. By activating (e.g., illuminating) a corresponding indicator 1902 of the transection pause indicator array 1900, the control system 820 can provide an indication of the number and relative location of each transection pause.

For example, an illustrative transection pause indicator array 1910 includes a single activated indicator 1902 that is located in the middle of the array 1910 to provide an indication that a single transection pause has been determined and is centrally located (which may correspond to the peak firing force 1410 of FIG. 14). Alternatively, an illustrative transection pause indicator array 1920 includes two activated indicators 1902 spaced apart from the central indicator 1902, which provides an indication that two transection pauses have been determined and are evenly spaced from the peak firing force 1410. In another embodiment, an illustrative transection pause indicator array 1930 includes three activated indicators 1902, which provides an indication that three transection pauses have been determined and their relative locations. Further, in another embodiment, an illustrative transection pause indicator array 1950 includes five activated indicators 1902, which provides an indication that five transection pauses have been determined and their relative locations.

Referring back to FIG. 12B, in some embodiments, the control system 820 may additionally or alternatively display an expected firing force profile to the operator of the robotic surgical system 800 in block 1244. The expected firing force profile may include indica of the number and relative positioning of each determined transection pause. In some embodiments, the displayed expected firing force profile may be similar to the transection profile 1500 shown in FIG. 15 and discussed above in regard to block 1226. Similar to transection profile 1500, the expected firing force profile may be displayed on the graphical user interface 1550 displayed to the operator on the display 812 of the control console 802.

After the control system 820 has communicated the indicia of the number (and location) of the determined transection pauses in block 1230 or if no communication is to occur, the method 1200 advances to block 1246. In block 1246, the control system 820 determines whether the operator of the robotic surgical system 800 desires to abort the firing of the surgical stapler 100. For example, the operator (e.g., surgeon 850) may decide to abort the firing of the surgical stapler 100 based on the total number of scheduled transection pause and/or their scheduled relative positioning. By aborting the firing process, the operator may, for example, adjust the grasping of the tissue by the end effector 110 and/or take other corrective measures to obtain a more desirable firing force profile, number of transection pauses, and/or location of transection pauses.

If, however, the operator (e.g., surgeon 850) decides to proceed with the firing of the end effector 110, the method 1200 advances to block 1248 in which the control system 820 awaits await activation of the firing by the operator, which may be provided by one of the input controls 814 of the control console 802. If an activation command is received, the method 1200 advances to block 1250 in which the control system 820 performs the firing phase of the surgical stapler 100. In doing so, the control system 820 controls the surgical stapler 100 to implement each of the determined transection pauses in the determined relative location of the firing force profile. In doing so, as discussed above, advancement of the knife 450 of the end effector 110 is halted during each transection pause. After the firing phase has been completed, the method 1200 loops back to block 1202 in which the control system 820 determines whether the jaw assembly 200 has again been positioned in the closed state and a subsequent firing phase may be initiated.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

### EXAMPLES

Example 1 includes a surgical system having a surgical stapler and a control system. The surgical stapler includes an end effector having a jaw assembly movable from an open state to a closed state to compress tissue captured within the jaw assembly. When in the closed state, the surgical stapler is operable to perform a firing phase to transect and staple the tissue captured within the jaw assembly. The control system is configured to control operation of the surgical stapler and is configured to determine a total amount of work completed by the jaw assembly to compress the tissue and determine a number of transection pauses to be performed by the end effector during the firing phase of the surgical stapler based on the determined total amount of work performed by the end effector to compress the tissue.

Example 2 includes the subject matter of Example 1, and wherein to determine the total amount of work performed by the jaw assembly includes to determine, while the jaw assembly compresses the tissue, an amount of torque of a motor configured to control the jaw assembly; determine, while the jaw assembly compresses the tissue, a rotational angle of the motor; and determine the total amount of work performed by the jaw assembly to compress the tissue based on the determined amount of torque of the motor and the rotational angle of the motor.

Example 3 includes the subject matter of any of Examples 1 or 2, and wherein the control system further includes a torque sensor configured to generate sensor data indicative of the torque of the motor and a rotary encoder configured to generate sensor data indicative of the rotational angle of the motor.

Example 4 includes the subject matter of any of Examples 1-3, and wherein the end effector includes a knife movable to transect the tissue compressed by the jaw assembly during the firing phase of the end effector. Additionally, movement of the knife is halted during each transection pause.

Example 5 includes the subject matter of any of Examples 1-4, and wherein to determine the number of transection pauses to be performed by the end effector during the firing phase of the surgical stapler includes to determine a number of reference work increments completed within the total amount of work completed by the jaw assembly to compress the tissue and to increment the number of transection pauses for each reference work increment completed.

Example 6 includes the subject matter of any of Examples 1-5, and wherein the reference work increment is 0.25 Joules.

Example 7 includes the subject matter of any of Examples 1-6, and wherein the control system is further configured to determine a position for each transection pause on a firing force profile of the end effector during the firing phase.

Example 8 includes the subject matter of any of Examples 1-7, and wherein the firing force profile of the end effector is based on a historical firing force profile of the end effector.

Example 9 includes the subject matter of any of Examples 1-8, and wherein to determine the position for each transection pause comprises to space each transection pause in reference to a peak firing force value of the firing force profile.

Example 10 includes the subject matter of any of Examples 1-9, and wherein to space each transection pause in reference to the peak firing force value comprises to evenly space each transection pause from the peak firing force value of the firing force profile.

Example 11 includes the subject matter of any of Examples 1-10, and wherein the control system is further configured to display a simulated transection profile to an operator of the surgical stapler prior to the firing phase of the surgical stapler, wherein the simulated transection profile is indicative of a position of a knife of the end effector throughout the firing phase and includes indicia of the number and position of each transection pause on the simulated transection profile, and wherein movement of the knife is halted during each transection pause.

Example 12 includes the subject matter of any of Examples 1-11, and wherein the control system is further configured to communicate indicia of the number of transection pauses to an operator of the surgical stapler.

Example 13 includes the subject matter of any of Examples 1-12, and wherein to communicate indicia of the number of transection pauses comprises to display a pause count indicator to the operator, wherein the pause count indicator is indicative of the number of transection pauses.

Example 14 includes the subject matter of any of Examples 1-13, and wherein the control system is further configured to accentuate the pause count indicator in response to the number of transection pauses changing.

Example 15 includes the subject matter of any of Examples 1-14, and wherein to communicate indicia of the number of transection pauses comprises to generate an audible feedback indicative of the number of transection pauses.

Example 16 includes the subject matter of any of Examples 1-15, and wherein to communicate indicia of the number of transection pauses comprises to generate a haptic feedback indicative of the number of transection pauses.

Example 17 includes the subject matter of any of Examples 1-16, and wherein to communicate indicia of the number of transection pauses comprises to display visual feedback on a graphical user interface. The visual feedback includes indicia of the number and location of each transection pause relative to each other.

Example 18 includes the subject matter of any of Examples 1-17, and wherein to display visual feedback comprises to display a transection pause indicator array, wherein the transection pause indicator array includes a plurality of indicators arranged in positions relative to each other and wherein activation of an indicator of the plurality of indicators is indicative of a transection pause at a location indicated by the corresponding indicator.

Example 19 includes the subject matter of any of Examples 1-18, and wherein to display visual feedback comprises to display an expected firing force profile graph having an ordinate axis of transection force and an abscissa axis of position of a knife of the end effector, and wherein the expected firing force profile graph includes indicia of the number and location of each transection pause.

Example 20 includes the subject matter of any of Examples 1-19, and wherein the control system is further configured to control the surgical stapler to perform the firing phase of the end effector, wherein to perform the firing phase includes to pause transection of the tissue by the end effector based on the number of transection pauses.

Example 21 includes a control system for controlling operation of a surgical stapler including an end effector having a jaw assembly moveable from an open state to a closed state to compress tissue captured within the jaw assembly. The control system includes a torque sensor configured to generate torque sensor data indicative of an amount of torque of a motor configured to control the jaw assembly of the surgical stapler; a rotary encoder configured to generate rotation sensor data indicative of the rotational angle of the motor; and a controller configured to (i) determine a total amount of work completed by the jaw assembly to compress the tissue based on the torque sensor data and the rotation sensor data and (ii) determine, based on the determined total amount of work performed by the end effector to compress the tissue, a number of transection pauses to be performed by the end effector during a firing phase of the surgical stapler to transect and staple the tissue captured within the jaw assembly.

Example 22 includes the subject matter of Example 21, and wherein to determine the number of transection pauses to be performed by the end effector during the firing phase of the surgical stapler comprises to determine a number of reference work increments completed within the total amount of work completed by the jaw assembly to compress the tissue and to increment the number of transection pauses for each reference work increment completed.

Example 23 includes the subject matter of any of Examples 21 or 22, and wherein the reference work increment is 0.25 Joules.

Example 24 includes the subject matter of any of Examples 21-23, and wherein the controller is further configured to determine a position for each transection pause on a firing force profile of the end effector during the firing phase.

Example 25 includes the subject matter of any of Examples 21-24, and wherein the firing force profile of the end effector is based on a historical firing force profile of the end effector.

Example 26 includes the subject matter of any of Examples 21-25, and wherein to determine the position for each transection pause comprises to space each transection pause in reference to a peak firing force value of the firing force profile.

Example 27 includes the subject matter of any of Examples 21-26, and wherein to space each transection pause in reference to the peak firing force value comprises to evenly space each transection pause from the peak firing force value of the firing force profile.

Example 28 includes the subject matter of any of Examples 21-27, and wherein the controller is further configured to display a simulated transection profile to an operator of the surgical stapler prior to the firing phase of the surgical stapler. The simulated transection profile is indicative of a position of a knife of the end effector throughout the firing phase and includes indicia of the number and position of each transection pause on the simulated transection profile, and wherein movement of the knife is halted during each transection pause.

Example 29 includes the subject matter of any of Examples 21-28, and wherein the control system is further configured to communicate indicia of the number of transection pauses to an operator of the surgical stapler.

Example 30 includes the subject matter of any of Examples 21-29, and wherein to communicate indicia of the number of transection pauses comprises to display a pause count indicator to the operator, wherein the pause count indicator is indicative of the number of transection pauses.

Example 31 includes the subject matter of any of Examples 21-30, and wherein the controller is further configured to accentuate the pause count indicator in response to the number of transection pauses changing.

Example 32 includes the subject matter of any of Examples 21-31, and wherein to communicate indicia of the number of transection pauses comprises to generate an audible feedback indicative of the number of transection pauses.

Example 33 includes the subject matter of any of Examples 21-32, and wherein to communicate indicia of the number of transection pauses comprises to generate a haptic feedback indicative of the number of transection pauses.

Example 34 includes the subject matter of any of Examples 21-33, and wherein to communicate indicia of the number of transection pauses comprises to display visual feedback on a graphical user interface, wherein the visual feedback includes indicia of the number and location of each transection pause relative to each other.

Example 35 includes the subject matter of any of Examples 21-34, and wherein to display visual feedback comprises to display a transection pause indicator array, wherein the transection pause indicator array includes a plurality of indicators arranged in positions relative to each other and wherein activation of an indicator of the plurality of indicators is indicative of a transection pause at a location indicated by the corresponding indicator.

Example 36 includes the subject matter of any of Examples 21-35, and wherein to display visual feedback comprises to display an expected firing force profile graph having an ordinate axis of transection force and an abscissa axis of position of a knife of the end effector, wherein the expected firing force profile graph includes indicia of the number and location of each transection pause.

Example 37 includes the subject matter of any of Examples 21-36, and wherein the controller is further configured to control the surgical stapler to perform the firing phase of the end effector, wherein to perform the firing phase includes to pause transection of the tissue by the end effector based on the number of transection pauses.

Example 38 includes a robotic manipulator having a robotic arm and a control system. The robotic arm includes an arm interface configured to mate with a tool interface of a surgical stapler to couple the surgical stapler to the robotic arm. The control system is configured to (i) control operation of the surgical stapler when the surgical stapler is coupled to the robotic arm to move a jaw assembly of an end effector of the surgical stapler from an open state to a closed state to compress tissue captured within the jaw assembly, (ii) determine a total amount of work completed by the jaw assembly to compress the tissue, and (iii) determine, based on the determined total amount of work performed by the end effector to compress the tissue, a number of transection pauses to be performed by the end effector during a firing phase of the surgical stapler to transect and staple the tissue captured within the jaw assembly.

Example 39 includes the subject matter of Example 38, and wherein to determine the total amount of work performed by the jaw assembly comprises to determine, while the jaw assembly compresses the tissue, an amount of torque of a motor of the robotic arm configured to control the jaw assembly via the arm interface; to determine, while the jaw assembly compresses the tissue, a rotational angle of the motor; and to determine the total amount of work performed by the jaw assembly to compress the tissue based on the determined amount of torque of the motor and the rotational angle of the motor.

Example 40 includes the subject matter of any of Examples 38 or 39, and wherein the control system includes a torque sensor configured to generate sensor data indicative of the torque of the motor and a rotary encoder configured to generate sensor data indicative of the rotational angle of the motor.

Example 41 includes the subject matter of any of Examples 38-40, and wherein to determine the number of transection pauses to be performed by the end effector of the surgical stapler during the firing phase of the surgical stapler comprises to determine a number of reference work increments completed within the total amount of work completed by the jaw assembly to compress the tissue and increment the number of transection pauses for each reference work increment completed.

Example 42 includes the subject matter of any of Examples 38-41, and wherein the reference work increment is 0.25 Joules.

Example 43 includes the subject matter of any of Examples 38-42, and wherein the control system is further configured to determine a position for each transection pause on a firing force profile of the end effector of the surgical stapler during the firing phase.

Example 44 includes the subject matter of any of Examples 38-43, and wherein the firing force profile of the end effector is based on a historical firing force profile of the end effector.

Example 45 includes the subject matter of any of Examples 38-44, and wherein to determine the position for each transection pause comprises to space each transection pause in reference to a peak firing force value of the firing force profile.

Example 46 includes the subject matter of any of Examples 38-45, and wherein to space each transection pause in reference to the peak firing force value comprises to evenly space each transection pause from the peak firing force value of the firing force profile.

Example 47 includes the subject matter of any of Examples 38-46, and wherein the control system is further configured to display a simulated transection profile to an operator of the surgical stapler prior to the firing phase of the surgical stapler, wherein the simulated transection profile is indicative of a position of a knife of the end effector of the surgical stapler throughout the firing phase and includes indicia of the number and position of each transection pause on the simulated transection profile, and wherein movement of the knife is halted during each transection pause.

Example 48 includes the subject matter of any of Examples 38-47, and wherein the control system is further configured to communicate indicia of the number of transection pauses to an operator of the surgical stapler.

Example 49 includes the subject matter of any of Examples 38-48, and wherein to communicate indicia of the number of transection pauses comprises to display a pause count indicator to the operator, wherein the pause count indicator is indicative of the number of transection pauses.

Example 50 includes the subject matter of any of Examples 38-49, and wherein the control system is further configured to accentuate the pause count indicator in response to the number of transection pauses changing.

Example 51 includes the subject matter of any of Examples 38-50, and wherein to communicate indicia of the number of transection pauses comprises to generate an audible feedback indicative of the number of transection pauses.

Example 52 includes the subject matter of any of Examples 38-51, and wherein to communicate indicia of the number of transection pauses comprises to generate a haptic feedback indicative of the number of transection pauses.

Example 53 includes the subject matter of any of Examples 38-52, and wherein to communicate indicia of the number of transection pauses comprises to display visual feedback on a graphical user interface, wherein the visual feedback includes indicia of the number and location of each transection pause relative to each other.

Example 54 includes the subject matter of any of Examples 38-53, and wherein to display visual feedback comprises to display a transection pause indicator array, wherein the transection pause indicator array includes a plurality of indicators arranged in positions relative to each other and wherein activation of an indicator of the plurality of indicators is indicative of a transection pause at a location indicated by the corresponding indicator.

Example 55 includes the subject matter of any of Examples 38-54, and wherein to display visual feedback comprises to display an expected firing force profile graph having an ordinate axis of transection force and an abscissa axis of position of a knife of the end effector, wherein the expected firing force profile graph includes indicia of the number and location of each transection pause.

Example 56 includes the subject matter of any of Examples 38-55, and wherein the control system is further configured to control the surgical stapler to perform the firing phase of the end effector, wherein to perform the firing phase includes to pause transection of the tissue by the end effector based on the number of transection pauses.

Example 57 includes a method for controlling operation of a surgical stapler. The method includes determining, by a control system, a total amount of work completed by a jaw assembly of an end effector of the surgical stapler to compress tissue captured within the jaw assembly; determining, by the control system, a number of transection pauses to be performed by the end effector during a firing phase of the surgical stapler based on the determined total amount of work performed by the end effector to compress the tissue; and controlling, by the control system, the surgical stapler to perform a firing phase of the end effector of the surgical stapler to transect and staple the tissue captured within the jaw assembly, wherein to perform the firing phase includes to pause transection of the tissue by the end effector based on the number of transection pauses.

Example 58 includes the subject matter of Example 57, and wherein determining the total amount of work performed by the jaw assembly comprises determining, while the jaw assembly compresses the tissue, an amount of torque of a motor configured to control the jaw assembly; determining, while the jaw assembly compresses the tissue, a rotational angle of the motor; and determining the total amount of work performed by the jaw assembly to compress the tissue based on the determined amount of torque of the motor and the rotational angle of the motor.

Example 59 includes the subject matter of any of Examples 57 or 58, and wherein determining the number of transection pauses to be performed by the end effector during the firing phase of the surgical stapler comprises determining a number of reference work increments completed within the total amount of work completed by the jaw assembly to compress the tissue and incrementing the number of transection pauses for each reference work increment completed.

Example 60 includes the subject matter of any of Examples 57-59, and wherein the reference work increment is 0.25 Joules.

Example 61 includes the subject matter of any of Examples 57-60, and further comprising determining, by the control system, a position for each transection pause on a firing force profile of the end effector during the firing phase.

Example 62 includes the subject matter of any of Examples 57-61, and wherein the firing force profile of the end effector is based on a historical firing force profile of the end effector.

Example 63 includes the subject matter of any of Examples 57-62, and wherein determining the position for each transection pause comprises spacing each transection pause in reference to a peak firing force value of the firing force profile.

Example 64 includes the subject matter of any of Examples 57-63, and wherein spacing each transection pause in reference to the peak firing force value comprises evenly spacing each transection pause from the peak firing force value of the firing force profile.

Example 65 includes the subject matter of any of Examples 57-64, and further comprising displaying, by the control system, a simulated transection profile to an operator of the surgical stapler prior to the firing phase of the surgical stapler, wherein the simulated transection profile is indicative of a position of a knife of the end effector throughout the firing phase and includes indicia of the number and position of each transection pause on the simulated transection profile, and wherein movement of the knife is halted during each transection pause.

Example 66 includes the subject matter of any of Examples 57-65, and further comprising communicating, by the control system, indicia of the number of transection pauses to an operator of the surgical stapler.

Example 67 includes the subject matter of any of Examples 57-66, and wherein communicating the indicia of the number of transection pauses comprises displaying a pause count indicator to the operator, wherein the pause count indicator is indicative of the number of transection pauses.

Example 68 includes the subject matter of any of Examples 57-67, and further comprising accentuating, by the control system, the pause count indicator in response to the number of transection pauses changing.

Example 69 includes the subject matter of any of Examples 57-68, and wherein communicating the indicia of the number of transection pauses comprises generating an audible feedback indicative of the number of transection pauses.

Example 70 includes the subject matter of any of Examples 57-69, and wherein communicating the indicia of the number of transection pauses comprises generating a haptic feedback indicative of the number of transection pauses.

Example 71 includes the subject matter of any of Examples 57-70, and wherein communicating the indicia of the number of transection pauses comprises displaying visual feedback on a graphical user interface, wherein the visual feedback includes indicia of the number and location of each transection pause relative to each other.

Example 72 includes the subject matter of any of Examples 57-71, and wherein displaying visual feedback comprises displaying a transection pause indicator array, wherein the transection pause indicator array includes a plurality of indicators arranged in positions relative to each other and wherein activation of an indicator of the plurality of indicators is indicative of a transection pause at a location indicated by the corresponding indicator.

Example 73 includes the subject matter of any of Examples 57-72, and wherein displaying visual feedback comprises displaying an expected firing force profile graph having an ordinate axis of transection force and an abscissa axis of position of a knife of the end effector, wherein the expected firing force profile graph includes indicia of the number and location of each transection pause.

## Claims

1. A surgical system (800) comprising:
a surgical stapler (100) including an end effector (110) having a jaw assembly (200) movable from an open state to a closed state to compress tissue captured within the jaw assembly (200), wherein, when in the closed state, the surgical stapler (100) is operable to perform a firing phase to transect and staple the tissue captured within the jaw assembly (200); and
a control system (820) configured to control operation of the surgical stapler (100), wherein the control system (820) is configured to determine a total amount of work completed by the jaw assembly (200) to compress the tissue and determine a number of transection pauses to be performed by the end effector (110) during the firing phase of the surgical stapler (100) based on the determined total amount of work performed by the end effector (110) to compress the tissue.

2. The surgical system (800) of claim 1, wherein to determine the total amount of work performed by the jaw assembly (200) comprises to:
determine, while the jaw assembly compresses the tissue, an amount of torque of a motor (930) configured to control the jaw assembly (200);
determine, while the jaw assembly (200) compresses the tissue, a rotational angle of the motor (930); and
determine the total amount of work performed by the jaw assembly (200) to compress the tissue based on the determined amount of torque of the motor (930) and the rotational angle of the motor (930).

3. The surgical system (800) of any of claims 1 or 2, wherein to determine the number of transection pauses to be performed by the end effector (110) during the firing phase of the surgical stapler (100) comprises to:
determine a number of reference work increments completed within the total amount of work completed by the jaw assembly (200) to compress the tissue; and
increment the number of transection pauses for each reference work increment completed.

4. The surgical system (800) of any of claims 1-3, wherein the control system (820) is further configured to determine a position for each transection pause on a firing force profile (1402) of the end effector (110) during the firing phase.

5. The surgical system (800) of claim 4, wherein to determine the position for each transection pause comprises to space each transection pause in reference to a peak firing force value of the firing force profile (1402).

6. The surgical system (800) of any of claims 1-5, wherein the control system (820) is further configured to display a simulated transection profile (1500) to an operator of the surgical stapler (100) prior to the firing phase of the surgical stapler (100), wherein the simulated transection profile (1500) is indicative of a position of a knife (450) of the end effector (110) throughout the firing phase and includes indicia of the number and position of each transection pause on the simulated transection profile (1500), and wherein movement of the knife (450) is halted during each transection pause.

7. The surgical system (800) of any of claims 1-6, wherein the control system (820) is further configured to communicate indicia of the number of transection pauses to an operator of the surgical stapler (100).

8. The surgical system (800) of claim 7, wherein to communicate indicia of the number of transection pauses comprises to display a pause count indicator (1600) to the operator, wherein the pause count indicator (1600) is indicative of the number of transection pauses.

9. The surgical system of claim 8, wherein the control system is further configured to accentuate the pause count indicator (1600) in response to the number of transection pauses changing.

10. The surgical system (800) of claim 7, wherein to communicate indicia of the number of transection pauses comprises to generate an audible or haptic feedback indicative of the number of transection pauses.

11. The surgical system (800) of claim 7, wherein to communicate indicia of the number of transection pauses comprises to display visual feedback on a graphical user interface (1550), wherein the visual feedback includes indicia of the number and location of each transection pause relative to each other.

12. The surgical system (800) of claim 11, wherein to display visual feedback comprises to display a transection pause indicator array (1800), wherein the transection pause indicator array (1800) includes a plurality of indicators (1802) arranged in positions relative to each other and wherein activation of an indicator (1802) of the plurality of indicators (1802) is indicative of a transection pause at a location indicated by the corresponding indicator (1802).

13. The surgical system (800) of any of claims 1-12, wherein the control system (820) is further configured to control the surgical stapler (100) to perform the firing phase of the end effector (110), wherein to perform the firing phase includes to pause transection of the tissue by the end effector (110) based on the number of transection pauses.

14. A method for controlling operation of a surgical stapler (100), the method comprising:
determining, by a control system (820), a total amount of work completed by a jaw assembly (200) of an end effector (110) of the surgical stapler (100) to compress tissue captured within the jaw assembly;
determining, by the control system (820), a number of transection pauses to be performed by the end effector (110) during a firing phase of the surgical stapler (100) based on the determined total amount of work performed by the end effector (110) to compress the tissue; and
controlling, by the control system (820), the surgical stapler (100) to perform a firing phase of the end effector (110) of the surgical stapler (100) to transect and staple the tissue captured within the jaw assembly (200), wherein to perform the firing phase includes to pause transection of the tissue by the end effector (110) based on the number of transection pauses.

15. The method of claim 14, further comprising determining, by the control system (820), a position for each transection pause on a firing force profile (1402) of the end effector (110) during the firing phase, wherein determining the position for each transection pause comprises spacing each transection pause in reference to a peak firing force value of the firing force profile (1402).
